# EUROPÄISCHE PATENTSCHRIFT

(11) **EP 0 824 915 B1**
(45) Veröffentlichungstag und Bekanntmachung des Hinweises auf die Patenterteilung: **03.11.2004**
(21) Anmeldenummer: 97113482.0
(22) Anmeldetag: 05.08.1997
(51) Int. Cl.: A61K 7/50, A61K 7/48, A61K 7/06, B01F 17/00

(54) **Verwendung von Glycoglycerolipiden als Tenside sowie kosmetische oder dermatologische Zubereitungen, solche Glycoglycerolipide enthaltend**
Use of glycoglycerolipids as surfactants and cosmetic or dermatological compositions containing them
Utilisation de glycoglycerolipides comme agents tensio-actifs et compositions cosmétiques ou dermatologiques les contenant

(30) Priorität: 23.08.1996 DE 19634020
(43) Veröffentlichungstag der Anmeldung: 25.02.1998
(73) Patentinhaber: Beiersdorf Aktiengesellschaft, 20245 Hamburg (DE)
(72) Erfinder: Schneider, Günther, Dr., 22607 Hamburg (DE); Scheel, Oliver, Dr., 22559 Hamburg (DE); Thiem, Joachim, Prof. Dr., 22391 Hamburg (DE)

(56) Entgegenhaltungen:
- EP-A- 0 512 270
- EP-A- 0 550 280
- EP-A- 0 551 674
- WO-A-96/25142
- WO-A-97/11707
- PATENT ABSTRACTS OF JAPAN vol. 13, no. 273 (C-609), 22. Juni 1989 & JP 01 067237 A (NISSHIN OIL MILLS LTD), 13. März 1989
- PATENT ABSTRACTS OF JAPAN vol. 95, no. 9, 31. Oktober 1995 & JP 07 149786 A (SAGAMI CHEM RES CENTER), 13. Juni 1995
- PATENT ABSTRACTS OF JAPAN vol. 11, no. 200 (C-431), 27. Juni 1987 & JP 62 022597 A (NISSHIN OIL MILLS LTD), 30. Januar 1987
- PATENT ABSTRACTS OF JAPAN vol. 8, no. 145 (C-232), 6. Juli 1984 & JP 59 053497 A (RAION KK), 28. März 1984
- PATENT ABSTRACTS OF JAPAN vol. 14, no. 535 (C-0781), 26. November 1990 & JP 02 225489 A (MECT CORP), 7. September 1990
- PATENT ABSTRACTS OF JAPAN vol. 16, no. 342 (C-0966), 24. Juli 1992 & JP 04 103595 A (KAO CORP), 6. April 1992
- PATENT ABSTRACTS OF JAPAN vol. 9, no. 134 (C-285), 8. Juni 1985 & JP 60 019716 A (TAKEDA YAKUHIN KOGYO KK), 31. Januar 1985

## Beschreibung

Die vorliegende Erfindung betrifft die Verwendung von neuen Glycoglycerolipiden auf dem Gebiete der kosmetischen sowie der pharmazeutischen Dermatologie. Insbesondere betrifft die vorliegende Erfindung Wirkstoffe und kosmetische oder dermatologische Zubereitungen, solche Wirkstoffkombinationen enthaltend. Insbesondere betrifft die vorliegende Erfindung Wirkstoffe, welche Tensideigenschaften besitzen und/oder welche die Hautfeuchtigkeit steigern. Ferner betrifft die Erfindung kosmetische und dermatologische Zubereitungen mit einem Gehalt an solchen Substanzen. In einer bevorzugten Ausführungsform betrifft die vorliegende Erfindung kosmetische Reinigungsmittel. In einer weiteren Ausführungsform betrifft die vorliegende Erfindung kosmetische Zubereitungen mit einem Gehalt an Substanzen welche die Hautfeuchtigkeit steigern.

Die äußerste Schicht der Epidermis, das Stratum corneum (Hornschicht), ist als wichtige Barriereschicht von besonderer Bedeutung u.a. für den Schutz vor Umwelteinflüssen und Austrocknung. Die Hornschicht wird im Kontakt mit der Umwelt ständig abgenutzt und muß deshalb ununterbrochen erneuert werden.

Ein heute in der Fachwelt weitverbreitetes Hautmodell faßt das Stratum corneum als Zwei-Komponenten-System, ähnlich einer Ziegelsteinmauer (Ziegelstein-Mörtel-Modell), auf. In diesem Modell entsprechen die Korneozyten (Hornzellen) den Ziegelsteinen, die kompliziert zusammengesetzte Lipidmembran in den Interzellularräumen entspricht dem Mörtel.

Außer ihrer Barrierewirkung gegen externe chemische und physikalische Einflüsse tragen die epidermalen Lipide auch zum Zusammenhalt der Hornschicht bei und haben Einfluß auf die Hautglätte. Im Gegensatz zu den Talgdrüsenlipiden, die keinen geschlossenen Film auf der Haut ausbilden, sind die epidermalen Lipide über die gesamte Hornschicht verteilt.

Das äußerst komplexe Zusammenwirken der feuchtigkeitsbindenden Substanzen und der Lipide der oberen Hautschichten ist für die Regulation der Hautfeuchte sehr wichtig. Daher enthalten Kosmetika in der Regel, neben ausgewogenen Lipidabmischungen und Wasser, wasserbindende Substanzen.

Neben der chemischen Zusammensetzung ist jedoch auch das physikalische Verhalten dieser Substanzen von Bedeutung. Daher ist die Entwicklung von sehr gut bioverträglichen Emulgatoren bzw. Tensiden mit flüssigkristallinen Eigenschaften wünschenswert. Damit formulierte Produkte unterstützen die flüssigkristalline Organisation der Interzellularlipide des Stratum Comeums und verbessern so die Barriereeigenschaften der Homschicht. Besonders vorteilhaft ist es, wenn deren Molekülbestandteile aus natürlicherweise in der Epidermis vorkommenden Substanzen bestehen.

JP-A-04 103 595 beschreibt Glycoglycerolipide und deren Verwendung als Ausgangsmaterial für Kosmetische Zusammensetzungen.

Es war überraschend und für den Fachmann nicht vorauszusehen, daß kosmetische oder pharmazeutische Zubereitungen, gekennzeichnet durch einen wirksamen Gehalt an einem oder mehreren pharmazeutisch und/oder kosmetisch unbedenklichen Glycoglycerolipiden gemäß Anspruch 1 den Nachteilen des Standes der Technik abhelfen.

Erfindungsgemäß werden das oder die Glycoglycerolipide gewählt aus der Gruppe der Substanzen der Struktur 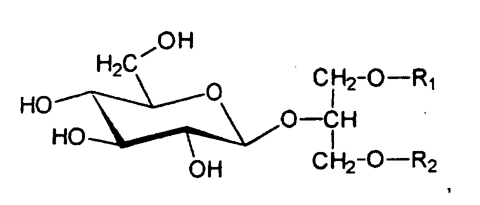 wobei R₁ und R₂ identisch sind und R₁ und/oder R₂ gewählt werden aus der Gruppe Octanoyl, Decanoyl, Dodecanoyl, Tetradecanoyl-, Hexadecanoyl-, Octadecanoyl-, Eicosanyl-.

Die den erfindungsgemäßen Hexosylglycerolipiden zugrundeliegenden Hexosen werden bevorzugt gewählt aus der Gruppe der Aldohexosen, gewöhnlich in ihrer pyranoiden Form, also Allo(pyrano)se, Altro(pyrano)se, Gluco(pyrano)se, Manno(pyrano)se, Gulo(pyrano)se, Ido(pyrano)se, Galakto(pyrano)se und Talo(pyrano)se. Vorteilhaft ist auch, die Hexosen zu wählen aus der Gruppe der Aminozucker.

D-Hexosylglycerolipide einzusetzen ist von Vorteil, gleichwohl können auch L-Hexosylglycerolipide gegebenenfalls vorteilhaft im Sinne der vorliegenden Erfindung eingesetzt werden.

Auch Hexosylglycerolipide, denen D- oder L-Ketohexosen zugrunde liegen, also Psicose, Fructose, Sorbose oder Tagatose, gewöhnlich in ihrer furanoiden Form vorliegend, können gegebenenfalls vorteilhaft im Sinne der vorliegenden Erfindung eingesetzt werden.

Besonders bevorzugte Hexosylglyceride sind 1,3-Diacylderivate des 2-O-β-D-Glucopyranosyl)-sn-glycerols.

Es war für den Fachmann daher nicht vorauszusehen gewesen, daß die erfindungsgemäßen Glycoglycerolipide bzw. kosmetische oder dermatologische Zubereitungen, diese enthaltend
- besser als feuchtigkeitsspendendes Agens wirken,
- besser gegen die Hautalterung wirken,
- besser als Tensid eignen und
- sich durch bessere Bioverträglichkeit auszeichnen würde
als die Wirkstoffe, Wirkstoffkombinationen und Zubereitungen des Standes der Technik.

Bevorzugt können Zwischenprodukte auf dem Wege zu den erfindungsgemäßen Wirkstoffen gemäß folgendem Reaktionsschema gewonnen werden (Reaktionsschema I):

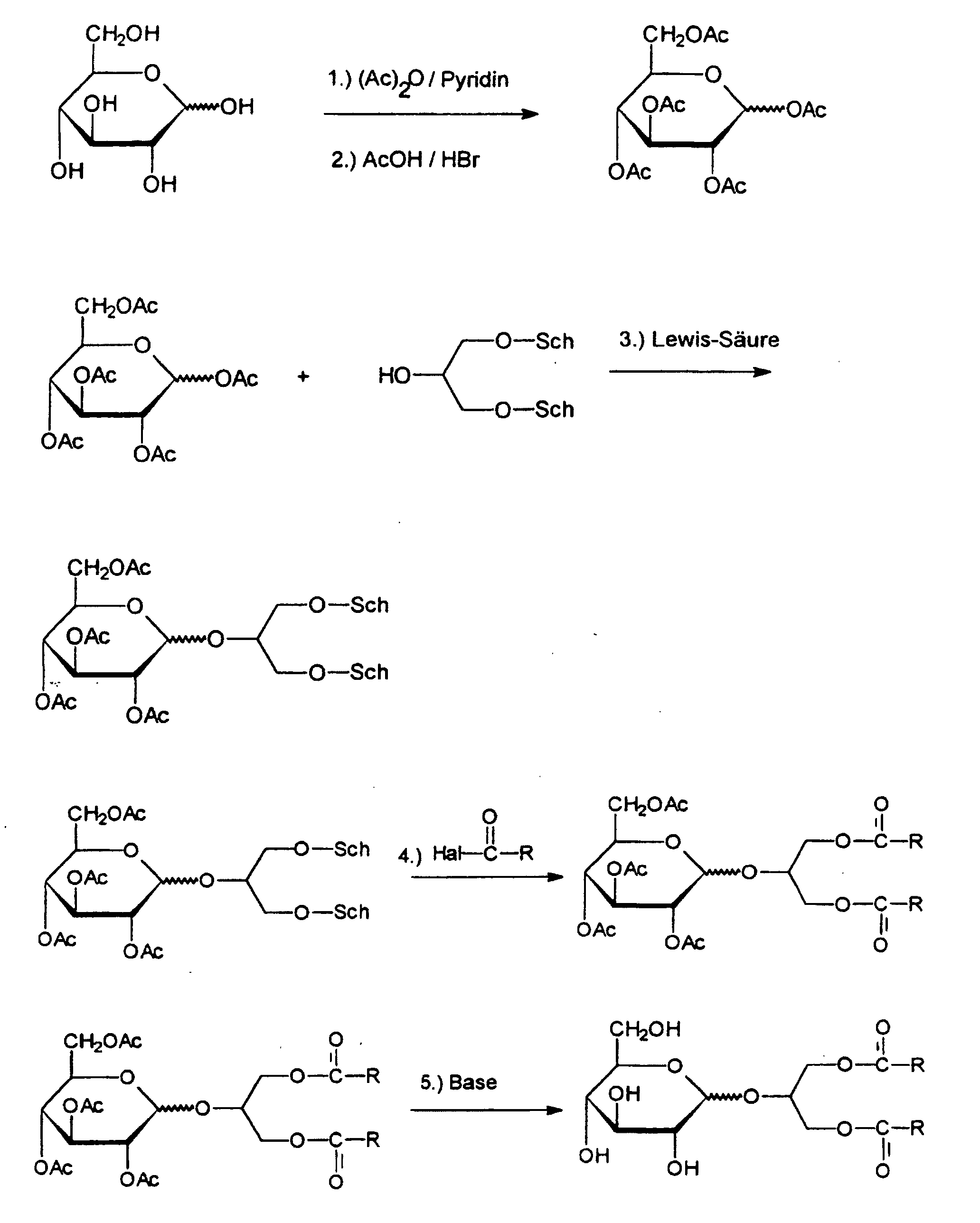

Die Bezeichnung Ac" steht dabei innerhalb eines Moleküls für Acetyl". Die Bezeichnung R" steht für einen den Acylresten R1 bzw. R2 zugrundeliegenden Alkylrest.

Reaktionsschritte 1) und 2) sind oft fakultativ, da eine Reihe acetylierter Bromzucker der Struktur 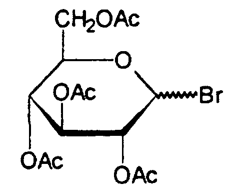 bekannt und käuflich zu erwerben sind.

Der Reaktionsschritt 3.) wird dabei vorteilhaft dergestalt durchgeführt, daß als Lewis-Säure Silbercarbonat gewählt wird und wobei als Lösungsmittel für das Zuckerderivat Methylenchlorid gewählt wird. Die Bezeichnung Sch" steht dabei für Schutzgruppe" wobei die beiden Schutzgruppen auch dergestalt miteinander verbunden sein können, daß sie mit dem Glycerylrest einen Ring bilden, beispielsweise verwirklicht im 5-Hydroxy-2-phenyl-1,3-dioxan:

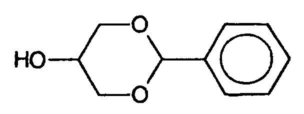

Die Umsetzung des im Reaktionsschritt 3.) gewonnenen Zwischenprodukts mit einem Acylhalogenid (vorteilhaft Acylchlorid) in einem nachgeschalteten Reaktionsschritt 4.), in basischem Milieu, bevorzugt unter Zugabe von Pyridin und durch basische Hydrolyse (beispielsweise mittels Methylamin in Methylalkohol, bevorzugt aber selektive hydrazinolytische Deacetylierung) in einem nachgeschalteten Reaktionsschritt 5.) führt zu den erfindungsgemäßen Glycoglycerolipiden.

Ein weiteres vorteilhaftes Reaktionsschema auf dem Wege zu den erfindungsgemäßen Wirkstoffen ist nachfolgend aufgeführt (Reaktionsschema II):

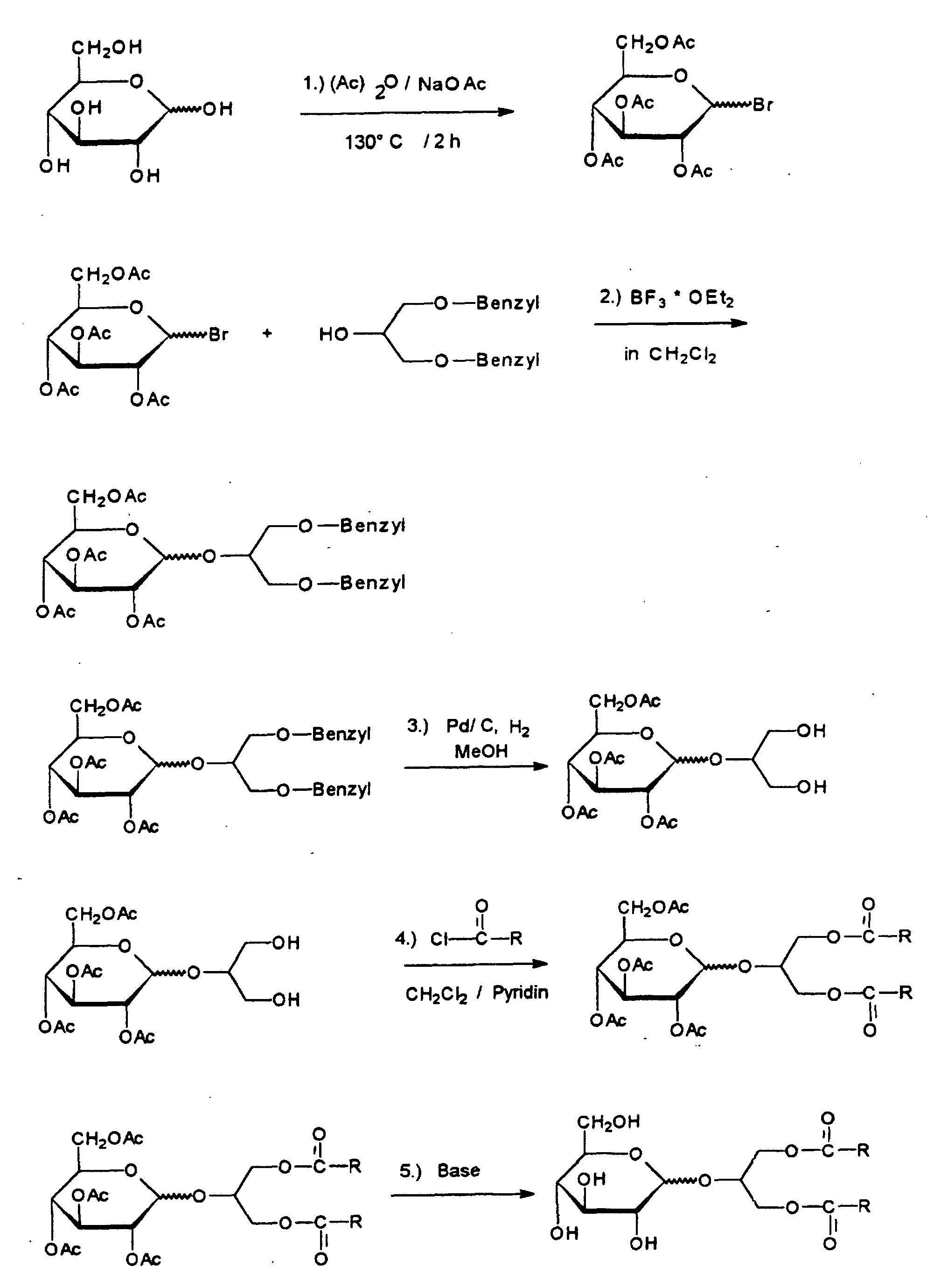

Die in Reaktionsschritt 5.) aufgeführte Base ist vorteilhaft N₂H₅OH, insbesondere in einem Gemisch aus 85% Ethylalkohol und Chloroform (1 : 1), wobei dieser Reaktionsschritt bevorzugt bei ca. 85° C über einen Zeitraum von ca. 1 Stunde durchgeführt wird.

Zu vorteilhaften Acetamidoglycoglyceriden bzw. Aminoglycoglyceriden gelangt man auf dem folgenden Wege (Reaktionsschema III):

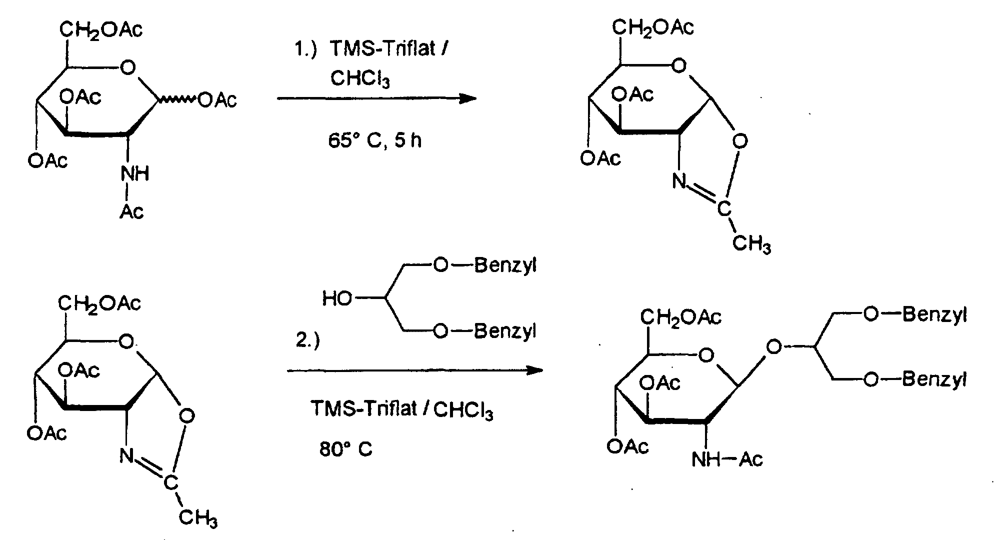

Ausgehend von dem in Reaktionsschritt 2.) gewonnenen Produkt kann nun entweder analog Reaktionsschema II weiter verfahren werden, wodurch Produkte beispielsweise gemäß der Strukturformel 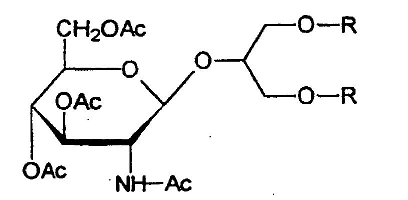 erhältlich sind.

Es ist aber auch, beispielsweise durch selektive Deacetylierung, möglich und vorteilhaft, die Acetamidofunktion in eine Aminofunktion umzuwandeln, wodurch Produkte beispielsweise gemäß der Strukturformel 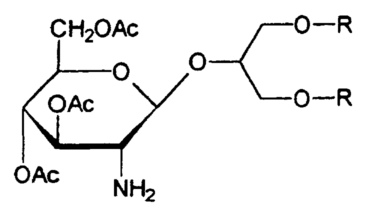 erhältlich sind.

Die Aufbereitung der erfindungsgemäßen Wirkstoffe erfolgt nach dem Fachmann geläufigen, üblichen Methoden.

Die erfindungsgemäßen kosmetischen oder dermatologischen Formulierungen können wie üblich zusammengesetzt sein und zur Behandlung, der Pflege und der Reinigung der Haut und/oder der Haare und als Schminkprodukt in der dekorativen Kosmetik dienen. Sie enthalten vorteilhaft 0,001 Gew.-% bis 95 Gew.-%, bevorzugt 0,01 Gew.-% bis 25 Gew.-%, insbesondere aber 0,25 Gew.-% bis 5 Gew.-%, bezogen auf das Gesamtgewicht der Zubereitungen, an den erfindungsgemäßen Glycoglycerolipiden.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsionen, beispielsweise vom Typ Wasser-in-Öl-in-Wasser (W/O/W), ein Gel, eine Hydrodispersion, einen festen Stift, eine Salbe oder auch ein Aerosol darstellen. Es ist auch vorteilhaft, die erfindungsgemäßen Glycoglycerolipide in verkapselter Form darzureichen, z.B. in Kollagenmatrices und anderen üblichen Verkapselungsmaterialien, z.B. als Celluloseverkapselungen, in Gelatine, Wachsmatrices oder liposomal verkapselt.

Es ist auch möglich und vorteilhaft im Sinne der vorliegenden Erfindung, die erfindungsgemäßen Glycoglycerolipiden in wäßrige Systeme bzw. Tensidzubereitungen zur Reinigung der Haut und der Haare einzufügen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Insbesondere können die erfindungsgemäßen Glycoglycerolipide auch mit Antioxidantien kombiniert werden.

Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den erfindungsgemäßen Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Die Lipidphase kann dabei vorteilhaft gewählt werden aus folgender Substanzgruppe:
- natürliche, synthetische und/oder partialsynthetische Öle, wie Triglyceride der Caprin- oder der Caprylsäure, vorzugsweise aber Rizinusöl;
- Fette, Wachse und andere natürliche synthetische und/oder partialsynthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.
- gesättigte Verbindungen wie Kohlenwasserstoffe natürlichen oder synthetischen Ursprungs (Vaseline, Squalan)

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugsweise ein Polyacrylat ist.

Als Treibmittel für erfindungsgemäße, aus Aerosolbehältern versprühbare Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Enthalten die erfindungsgemäßen Emulsionen UVB-Filtersubstanzen, können diese öllöslich oder wasserlöslich sein. Erfindungsgemäß vorteilhafte öllösliche UVB-Filter sind z.B.:
- 3-Benzylidencampher-Derivate, vorzugsweise 3-(4-Methylbenzyliden)campher, 3-Benzylidencampher;
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester, - 2,4,6-Trianilino-(p-carbo-2'-ethyl-1'-hexyloxy) -1,3,5-triazin.

Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UVB-Filter, die in Kombination mit den erfindungsgemäßen Glycoglycerolipiden verwendet werden können, soll selbstverständlich nicht limitierend sein.

Es kann auch von Vorteil sein, erfindungsgemäße Glycoglycerolipide mit UVA-Filtern zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Gegenstand der Erfindung ist auch die Verwendung einer Kombination der erfindungsgemäßen Glycoglycerolipide mit mindestens einem UVA-Filter als Antioxidans bzw. die Verwendung einer Kombination der erfindungsgemäßen Glycoglycerolipide mit mindestens einem UVA-Filter als Antioxidans in einer kosmetischen oder dermatologischen Zubereitung.

Gegenstand der Erfindung ist auch die Verwendung einer Kombination der erfindungsgemäßen Glycoglycerolipide mit mindestens einem UVA-Filter und mindestens einem UVB-Filter als Antioxidans bzw. die Verwendung einer Kombination der erfindungsgemäßen Glycoglycerolipide mit mindestens einem UVA-Filter und mindestens einem UVB-Filter als Antioxidans in einer kosmetischen oder dermatologischen Zubereitung.

Kosmetische und dermatologische Zubereitungen mit einem wirksamen Gehalt an erfindungsgemäßen Glycoglycerolipiden können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid.

Auch diese Kombinationen von UVA-Filter und Pigment bzw. Zubereitungen, die diese Kombination enthalten, sind Gegenstand der Erfindung. Es können die für die vorstehenden Kombinationen genannten Mengen verwendet werden.

Bei kosmetischen und dermatologischen Zubereitungen zum Schutze der Haare vor UV-Strahlen gemäß der Erfindung handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Die kosmetischen und dermatologischen enthalten Wirkstoffe und Hilfsstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Verdickungsmittel, Emulgatoren, Fette, Öle, Wachse, organische Lösungsmittel, Bakterizide, Parfüme, Farbstoffe oder Pigmente, deren Aufgabe es ist, die Haare oder die kosmetische oder dermatologische Zubereitung selbst zu färben, Elektrolyte, Substanzen gegen das Fetten der Haare.

Unter Elektrolyten im Sinne der vorliegenden Erfindung sind wasserlösliche Alkali-, Ammonium-, Erdalkali- (unter Einbeziehung des Magnesiums) und Zinksalze anorganischer Anionen und beliebige Gemische aus solchen Salzen zu verstehen, wobei gewährleistet sein muß, daß sich diese Salze durch pharmazeutische oder kosmetische Unbedenklichkeit auszeichnen.

Die erfindungsgemäßen Anionen werden bevorzugt gewählt aus der Gruppe der Chloride, der Sulfate und Hydrogensulfate, der Phosphate, Hydrogenphosphate und der linearen und cyclischen Oligophosphate sowie der Carbonate und Hydrogencarbonate.

Kosmetische Zubereitungen, die ein Hautreinigungsmittel oder Shampoonierungsmittel darstellen, enthalten vorzugsweise mindestens eine zusätzliche anionische, nichtionische oder amphotere oberflächenaktive Substanz, oder auch Gemische aus solchen Substanzen, erfindungsgemäße Glycoglycerolipide im wäßrigen Medium und Hilfsmittel, wie sie üblicherweise dafür verwendet werden. Die zusätzliche oberflächenaktive Substanz bzw. die Gemische aus diesen Substanzen können in einer Konzentration zwischen 1 Gew.-% und 50 Gew.-% in dem Shampoonierungsmittel vorliegen.

Liegen die kosmetischen oder dermatologischen Zubereitungen in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um wäßrige oder wäßrig-alkoholische Lösungen, die gegebenenfalls zusätzliche oberflächenaktive Substanzen enthalten, deren Konzentration zwischen 0,1 und 10 Gew.-%, vorzugsweise zwischen 0,2 und 5 Gew.-%, liegen kann.

. Diese kosmetischen oder dermatologischen Zubereitungen können auch Aerosole mit den üblicherweise dafür verwendeten Hilfsmitteln darstellen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält mindestens ein kationisches, anionisches, nicht-ionisches oder amphoteres Polymer oder auch Gemische derselben, sowie erfindungsgemäße Wirkstoffkombinatonen in wirksamer Konzentration. Die Menge der verwendeten Polymeren liegt z.B. zwischen 0,1 und 10 Gew.-%, bevorzugt zwischen 0,1 und 3 Gew.-%.

Kosmetische Zubereitungen zur Behandlung und Pflege der Haare, die erfindungsgemäße Glycosylglyceride enthalten, können als Emulsionen vorliegen, die vom nichtionischen oder anionischen Typ sind. Nicht-ionische Emulsionen enthalten neben Wasser Öle oder Fettalkohole, die beispielsweise auch polyethoxyliert oder polypropoxyliert sein können, oder auch Gemische aus den beiden organischen Komponenten. Diese Emulsionen enthalten gegebenenfalls kationische oberflächenaktive Substanzen.

Erfindungsgemäß können kosmetische Zubereitungen zur Behandlung und Pflege der Haare als Gele vorliegen, die neben einem wirksamen Gehalt an erfindungsgemäßen Glycoglycerolipide und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z.B. Aluminiumsilikate wie beispielsweise Bentonite, oder ein Gemisch aus Polyethylenglykol und Polyethylenglycolstearat oder - distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Vorzugsweise beträgt die Menge der erfindungsgemäßen Glycoglycerolipide in einem für die Haare bestimmten Mittel 0,05 Gew.-% bis 10 Gew.-%, insbesondere 0,5 Gew.-% bis 5 Gew.-%, bezogen auf das Gesamtgewicht des Mittels.

Erfindungsgemäße wäßrige kosmetische Reinigungsmittel oder für die wäßrige Reinigung bestimmte wasserarme oder wasserfreie Reinigungsmittelkonzentrate können anionische, nichtionische und/oder amphotere Tenside enthalten, beispielsweise herkömmliche Seifen, z.B. Fettsäuresalze des Natriums, Alkylsulfate, Alkylethersulfate, Alkan- und Alkylbenzolsulfonate, Sulfoacetate, Sulfobetaine, Sarcosinate, Amidosulfobetaine, Sulfosuccinate, Sulfobernsteinsäurehalbester, Alkylethercarboxylate, Eiweiß-Fettsäure-Kondensate, Alkylbetaine und Amidobetaine, Fettsäurealkanolamide und/oder Polyglycolether-Derivate.

Kosmetische Zubereitungen, die kosmetische Reinigungszubereitungen für die Haut darstellen, können in flüssiger oder fester Form vorliegen. Sie enthalten neben erfindungsgemäßen Glycoglycerolipiden vorzugsweise mindestens eine anionische, nichtionische oder amphotere oberflächenaktive Substanz oder Gemische daraus, gewünschtenfalls einen oder mehrere Elektrolyten und Hilfsmittel, wie sie üblicherweise dafür verwendet werden. Die oberflächenaktive Substanz kann in einer Konzentration zwischen 1 und 94 Gew.-% in den Reinigungszubereitungen vorliegen, bezogen auf das Gesamtgewicht der Zubereitungen.

Kosmetische Zubereitungen, die ein Shampoonierungsmittel darstellen, enthalten neben einem wirksamen Gehalt an erfindungsgemäßen Glycoglycerolipide vorzugsweise mindestens eine anionische, nicht-ionische oder amphotere oberflächenaktive Substanz oder Gemische daraus, gegebenenfalls einen erfindungsgemäßes Elektrolyten und Hilfsmittel, wie sie üblicherweise dafür verwendet werden. Die oberflächenaktive Substanz kann in einer Konzentration zwischen 1 Gew.-% und 94 Gew.-% in dem Shampoonierungsmittel vorliegen.

Die erfindungsgemäßen Zusammensetzungen enthalten außer den vorgenannten Tensiden Wasser und gegebenenfalls die in der Kosmetik üblichen Zusatzstoffe, beispielsweise Parfüm, Verdicker, Farbstoffe, Desodorantien, antimikrobielle Stoffe, rückfettende Agentien, Komplexierungs- und Sequestrierungsagentien, Perlglanzagentien, Pflanzenextrakte, Vitamine, Wirkstoffe und dergleichen.

Die nachfolgenden Beispiele sollen die vorliegende Erfindung Verdeutlichen.

### Beispiel 1 (O/W-Emulsion):

| | Gew.-% |
|---|---|
| Glycerinmonostearat | 2,00 |
| 1,3-Di-O-hexadecyl-2-O-D-(glucopyranosyl)-sn-glycerin | 2,00 |
| Paraffinöl, subliquidum | 11,00 |
| Octyldodecanol | 4,00 |
| Hydrierte Kokosfettsäureglyceride | 1,00 |
| Cyclomethicone | 1,00 |
| Carbomer | 0,15 |
| Glycerin | 2,00 |
| Tocopherylacetat | 0,50 |
| Parfüm, Konservierungsmittel, NaOH, | |
| Farbstoffe, Antioxidantien etc. | q.s. |
| Wasser | ad 100,00 |

### Beispiel 2 (O/W-Emulsion):

| | Gew.-% |
|---|---|
| Stearinsäure | 1,50 |
| Sorbitanmonostearat | 0,50 |
| 1,3-Di-O-hexadecyl-2-O-D-(glucopyranosyl)-sn-glycerin | 2,00 |
| Myristylalkokol | 1,50 |
| Glycerinmonostearat | 0,50 |
| Paraffinöl, subliquidum | 10,00 |
| Dimethicone | 1,00 |
| Octyldodecanol | 2,00 |
| Hydrierte Kokosfettsäureglyceride | 0,50 |
| Carbomer | 0,10 |
| Serin | 0.50 |
| Glycerin | 2,00 |
| Tocopherylacetat | 0,50 |
| Farbstoffe, Antioxidantien etc. | q.s. |
| Wasser | ad 100,00 |

### Beispiel 3 (O/W-Emulsion):

| | Gew.-% |
|---|---|
| Sorbitanmonostearat | 2,00 |
| Laurylmethicone Copolyol | 0,35 |
| Cetylmethicone Copolyol | 0,15 |
| 1,3-Di-O-hexadecyl-2-O-D-(glucopyranosyl)-sn-glycerin | 2,00 |
| Paraffinöl, subliquidum | 10,00 |
| Octyldodecanol | 4,00 |
| Hydrierte Kokosfettsäureglyceride | 1,00 |
| Cyclomethicone | 1,00 |
| Dimethicone | 1,00 |
| Carbomer | 0,15 |
| Glycerin | 2,00 |
| Tocopherylacetat | 0,50 |
| Farbstoffe, Antioxidantien etc. | q.s. |
| Wasser | ad 100,00 |

### Beispiel 4 (Shampoo)

| | Gew.-% |
|---|---|
| Natrium Laurylethersulfat (27,5 %) | 25,00 |
| 1,3-Di-O-dodecyl-2-O-D-(glucopyranosyl)-sn-glycerin | 4,00 |
| Farbstoffe, Konservierungsmittel, Perlglanzmittel | q.s. |
| Wasser | ad 100,00 |

### Beispiel 5 (Shampoo)

| | Gew.-% |
|---|---|
| Alkylpolyglucose (50 %) | 16,00 |
| 1,3-Di-O-dodecyl-2-O-D-(glucopyranosyl)-sn-glycerin | 4,00 |
| Farbstoffe, Konservierungsmittel, Perlglanzmittel | q.s. |
| Wasser | ad 100,00 |

### Beispiel 6 (Shampoo)

| | Gew.-% |
|---|---|
| Natrium Laurylethersulfat (27,5 %) | 14,00 |
| Alkylpolyglucose ( 50%) | 8,00 |
| 1,3-Di-O-dodecyl-2-O-D-(glucopyranosyl)-sn-glycerin | 4,00 |
| Farbstoffe, Konservierungsmittel, Perlglanzmittel | q.s |
| Wasser | ad 100,00 |

## Patentansprüche

1. Kosmetische oder pharmazeutische Zubereitungen, **gekennzeichnet durch** einen wirksamen Gehalt an einem oder mehreren pharmazeutisch und/oder kosmetisch unbedenklichen Glycoglycerolipiden, wobei das oder die Glycoglycerolipide gewählt wird oder werden aus der Gruppe der Substanzen der Struktur 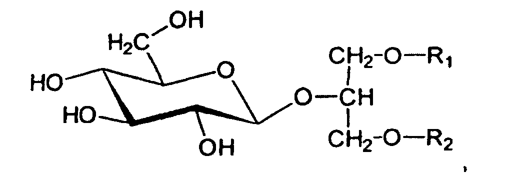 wobei R₁ und R₂ identisch sind und gewählt werden aus der Gruppe Octanoyl, Decanoyl, Dodecanoyl, Tetradecanoyl-, Hexadecanoyl-, Octadecanoyl-, Eicosanyl-.

2. Kosmetische oder pharmazeutische Zubereitungen nach Anspruch 1, **dadurch gekennzeichnet, daß** sie 0,01 Gew.-% bis 25 Gew.-%, insbesondere 0,25 Gew.-% bis 5 Gew.-% an Glycoglycerolipiden enthalten, bezogen auf das Gesamtgewicht der Zubereitungen.

3. Verwendung von Glycoglycerolipiden nach Anspruch 1 als die Hautfeuchtigkeit steigernde Agenzien, als oberflächenaktive und/oder grenzflächenaktive Wirkstoffe, insbesondere als waschaktive Tenside oder Emulgatoren.

## Claims

1. Cosmetic or pharmaceutical preparations **characterized by** an effective content of one or more pharmaceutically and/or cosmetically acceptable glycoglycerolipids, wherein the glycoglycerolipid or glycoglycerolipids is or are selected from the group of substances of the structure 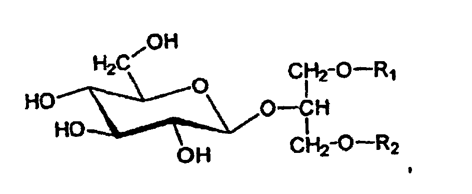 in which R₁ and R₂ are identical and are selected from the octanoyl, decanoyl, dodecanoyl, tetradecanoyl-, hexadecanoyl-, octadecanoyl- and eicosanyl-.

2. Cosmetic or pharmaceutical preparations according to Claim 1, **characterized in that** they contain 0.01% by weight to 25% by weight, especially 0.25% by weight to 5% by weight, of glycoglycerolipids, based on the total weight of the preparations.

3. Use of glycoglycerolipids according to Claim 1 as agents for increasing the moisture content of the skin, as surface-active substances and especially as washing-active surfactants or emulsifiers.

## Revendications

1. Préparations cosmétiques ou pharmaceutiques **caractérisées par** une teneur active en un ou plusieurs glycoglycérolipides pharmaceutiquement et/ou cosmétiquement acceptables, le ou les glycoglycérolipides étant choisi(s) parmi le groupe des substances de structure 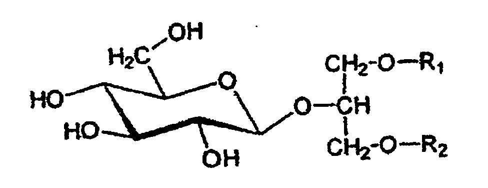 où R₁ et R₂ sont identiques et sont choisis parmi le groupe des octanoyle, décanoyle, dodécanoyle, tétradécanoyle, hexadécanoyle, octadécanoyle, éicosanyle.

2. Préparations cosmétiques ou pharmaceutiques selon la revendication 1 **caractérisées en ce qu'**elles contiennent 0,01 à 25% en poids, en particulier 0,25 à 5% en poids de glycoglycérolipides par rapport au poids total des préparations.

3. Utilisation de glycoglycérolipides selon la revendication 1 comme agents augmentant l'humidité de la peau, comme substances tensioactives, en particulier comme tensioactifs détergents ou émulsifiants.
